# EUROPEAN PATENT SPECIFICATION

(11) **EP 4 269 410 B1**
(45) Date of publication and mention of the grant of the patent: **03.06.2026**
(21) Application number: 22748878.0
(22) Date of filing: 20.01.2022
(51) Int. Cl.: A61K 31/4745, C07D 471/04, A61K 36/534, A61P 15/00

(54) **NEW USE OF PQQ AND DERIVATIVE THEREOF**
NEUE VERWENDUNG VON PQQ UND DERIVAT DAVON
NOUVELLE UTILISATION DE PQQ ET DE SON DÉRIVÉ

(30) Priority: 02.02.2021 CN 202110140223
(43) Date of publication of application: 01.11.2023
(73) Proprietor: Guangzhou Hutongyouwu Biotechnology Co., Ltd, Guangzhou, Guangdong 510630 (CN)
(72) Inventor: GUO, Ling, Guangzhou, Guangdong 510630 (CN); ZHANG, Xiaowen, Guangzhou, Guangdong 510630 (CN)
(74) Representative: Santarelli
(86) International application number: PCT/CN2022/072881
(87) International publication number: WO 2022/166599

(56) References cited:
- WO-A1-2018/065076
- WO-A1-2020/262325
- CN-A- 102 665 707
- CN-A- 104 856 996
- CN-A- 105 853 502
- RUCKER, R. ET AL.: "Potential Physiological Importance of Pyrroloquinoline Quinone", ALTERNATIVE MEDICINE REVIEW, vol. 14, no. 3, 31 December 2009 (2009-12-31), XP055325799

## Description

### Technical Field

The present invention relates to pyrroloquinoline quinone for new therapeutic use according to the appended claims.

### Background of the Invention

Dysmenorrhea is one of the most common gynecological symptoms, which refers to patients with lower abdominal pain, heaviness, and backache or other discomfort before and after menstruation or during menstruation, and the quality of life is seriously affected by the symptoms. Dysmenorrhea can be divided into two categories: primary dysmenorrhea and secondary dysmenorrhea. Primary dysmenorrhea refers to dysmenorrhea without organic lesions in reproductive organs; secondary dysmenorrhea refers to dysmenorrhea caused by pelvic organic diseases. The reasons of dysmenorrhea is not fully understood. It is generally believed that the pathogenesis and treatment plan of secondary dysmenorrhea are different from those of primary dysmenorrhea. Dysmenorrhea is often manifested in various ways of pain: lower abdominal pain, lower abdominal distension, pain in the anus, pain during sexual intercourse, etc.

Most mammals do not have regular menstrual cycles and mainly experience cryptomenorrhea, which is hardly comparable to the secondary menstrual pain indicators in humans. Although some primates also have menstruation, but no secondary dysmenorrhea is observed. The lack of a practical animal model available of dysmenorrhea for the development of drugs, which limits the progress of drug development. Existing papers also show that experimental results in animals or cell models of dysmenorrhea are often ineffective when applied to humans. Such as Durak, Yildirim, et al. ("Effect of vitamin C on the growth of experimentally induced endometriotic cysts." Journal of Obstetrics and Gynaecology Research 39.7 (2013): 1253-1258) indicated that vitamin C supplementation significantly reduced endometriotic cyst volume and weight in a dose-dependent manner. However, it is well known that vitamin C supplementation does not have any alleviating effect on human dysmenorrhea, let alone a therapeutic effect.

There are no very effective drugs for treating dysmenorrhea currently. Traditional drugs for treating dysmenorrhea mainly include hormonal drugs. However, treatment with hormonal or hormone-like drugs is associated with serious side effects, including but not limited to infertility, which limits its scope of application.

Pyrroloquinoline Quinone (PQQ) is a tricarboxylic acid quinone compound, CAS No. 72909-34-3, IUPAC Name: 4,5-dioxo-1H-pyrrolo[2,3-f]quinoline-2,7,9-tricarboxylic acid. PQQ is widely present in various foods. The content of PQQ in foods is about 3.65-61 ng/g, in parsley and green peppers in vegetables, kiwi and papaya in fruits, green tea and Oolong tea in drinks, tofu that people often eat, the content of PQQ is about 30ng/g. Previous studies have shown that PQQ can stimulate the speed of growth of cells of microorganisms, plants, animals and human; remove excess free radicals in body and protect body from oxidative damage; accelerate the oxidation of acetaldehyde to acetic acid to reduce the content of acetaldehyde in body, thereby it is expected to reduce the toxic damage to liver caused by alcohol consumption. CN110870866A discloses the application of pyrroloquinoline quinone in the preparation of medicines for preventing and treating acute altitude sickness and acute altitude hypoxia injury; CN110151764A discloses that pyrroloquinoline quinone can reduce lipopolysaccharide-induced animal fibroblasts and intestinal inflammation; CN106265730A discloses Application of pyrroloquinoline quinone (PQQ) in reversing tumor multidrug resistance; CN105963297A discloses the application of pyrroloquinoline quinone in adjuvant therapy after chemotherapy; CN103191115A discloses the application of pyrroloquinoline quinone in the treatment and/or alleviation of diabetic foot. WO2020/262325 discloses the use of PQQ for the treatment of menstrual disorders related to irregularities in the cycle of ovulation. WO2018/065076 discloses estetrol for use in treating primary dysmenorrhea.

### Technical solutions

The scope of protection of the present invention is defined in the appended set of claims.

A first aspect of the present invention provides:
Pyrroloquinoline quinone or derivatives thereof for use in a method of preventing, alleviating or treating a disease selected from:
- primary dysmenorrhea; and
- menstrual abnormalities selected from an abnormal increase in the amount of menstrual bleeding, and/or an abnormal increase in the duration of menstrual bleeding,
wherein said derivative of pyrroloquinoline quinone is a pharmaceutically or food acceptable salt, C2-C6 ester, amide, hydrate, crystal, co-crystal or solvate.

In some embodiments, said abnormal increase in the amount of menstrual bleeding refers to the amount of menstrual bleeding is 50% greater than the amount of a healthy woman.

In some embodiments, said abnormal increase in the duration of menstrual bleeding refers to the duration of menstrual bleeding is no less than 8 days.

In some embodiments, the oral dosage based on pyrroloquinoline quinone is:
about 5 mg/day to about 10 mg/day for preventing of primary dysmenorrhea;
about 15 mg/day to about 20 mg/day for alleviating or treating mild dysmenorrhea;
about 20 mg/day to about 30 mg/day for alleviating or treating moderate dysmenorrhea;
about 25 mg/day to about 100 mg/day for alleviating or treating severe dysmenorrhea;
about 20 mg/day to about 50 mg/day for alleviating or treating menstrual abnormalities.

In some embodiments, said composition is a food, a nutraceutical or a medicine.

In some embodiments, said composition further comprising pharmaceutically or food acceptable excipients.

In some embodiments, said excipient is selected from at least one of carriers, solvents, antioxidants, and preservatives.

In some embodiments, at least one of the following benefits is achieved by the patient after the preventing, alleviating or treating:
1) pregnant able;
2) alleviation of dysmenorrhea, preferably the average score of menstrual dysmenorrhea pain index drops by more than 1 point or the pain level drops by one grade;
3) the amount of menstrual bleeding is reduced to no more than 50%, 40%, 30% of the normal menstrual bleeding of women, preferably 10%, and more preferably reduced to the normal average menstrual bleeding;
4) the duration of menstrual bleeding is shortened to no longer than 10 days, preferably to no longer than 9 days, 8 days, or 7 days.

The pain index or pain level is determined according to WHO standards.

### Beneficial effect

The inventors found PQQ is effective in preventing, alleviating or treating patient with primary dysmenorrhea, secondary dysmenorrhea and menstrual abnormalities accidentally, wherein the tratement of secondary dysmenorrhea is mentioned for illustrative purposes only
In some embodiments, after being treated with PQQ, at least one of the following effects is achieved for patients: 1. the dysmenorrhea alleviation rate is 100%; 2. the average duration of a menstrual period is reduced from about 2 weeks to about 1 week, and is returned to the normal average duration of a menstrual period; 3. the amount of menstrual blood in a menstrual period is reduced by more than 30% on average, and is substantially returned to the normal average level; and 4. two patients suffering from years of infertility caused by secondary dysmenorrhea are pregnant within a treatment period of 3 months.

### Brief description of the Drawings

Figure 1 is the changes of the dysmenorrhea index (VAS score) in the treatment of secondary dysmenorrhea during treatment (not according to the claimed invention);
Figure 2 shows the changes of menstrual duration in patients with abnormal menstruation (irregular menstruation) during treatment;
Figure 3 shows the changes in the consumption of sanitary napkins of patients with abnormal menstruation (irregular menstruation) during treatment.

### Detailed Description of Embodiments

Pharmaceutical or food acceptable derivatives of the compound refer to simple derivatives thereof, especially the lower esters, lower ethers, lower alkyl substituents, pharmaceutically acceptable salts, and lower amides which the number of carbon atoms is 1 to 6, preferably 2 to 6, and a derivative achieved by condensation of a carboxylic acid, alcohol, or amine of 2 to 4 with the parent compound. In particular, the general formula of the derivative of pyrroloquinoline quinone is as following:

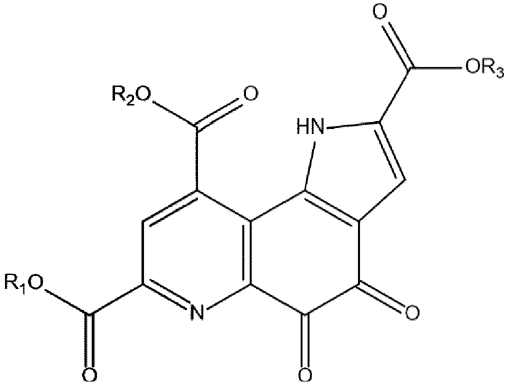

In the formula, R₁ to R₃ are the same or different, and are selected from H, C1 to C6 hydrocarbon groups, natural amino acid residues, Na, K, Li and other pharmaceutically acceptable groups. The derivatives of pyrroloquinoline quinone according to the invention are defined in the appended claims.

Pharmaceutically acceptable salts of the compounds can be synthesized from parent compounds by conventional chemical methods, such as those in Pharmaceutical Salts: Properties, Selection, and Use, P. Heinrich Stahl (Editor), Camille G. Wermuth (Editor), ISBN: 3-90639-026-8, Hardcover, 388 pages, August 2002. In general, these salts are prepared by the reaction of the free base and acid of the compound in water or an organic solvent or a mixture of the two; Typically, nonaqueous media such as ether, ethyl acetate, ethanol, isopropanol or acetonitrile are used.

Acid addition salts can be made by various acids (inorganic and organic acids). Embodiments of acid addition salts include a salt made by a compounds reacted with an acid, said acid is selected from a group consist of acetic acid, 2,2-dichloroacetic acid, adipic acid, alginic acid, ascorbic acid , L-aspartic acid, benzenesulfonic acid, benzoic acid, 4-acetylamino benzoic acid, butyric acid, (+)-camphoric acid, camphor sulfonic acid, (+)-(1S)-camphor-10-sulfonic acid, capric acid, hexanoic acid, octanoic acid, cinnamic acid, citric acid, cyclamic acid, dodecylsulfuric acid, ethane-1,2-disulfonic acid, ethanesulfonic acid, 2-hydroxyethanesulfonic acid, formic acid, fumaric acid, galactonic acid, gentisic acid, glucoheptonic acid, D-gluconic acid, glucuronic acid, glutamic acid , α-ketoglutaric acid, glycolic acid, hippuric acid, hydrobromic acid, hydrochloric acid, hydroiodic acid, isethionic acid, (+)-L-lactic acid, (±)-DL-lactic acid, lactobionic acid, maleic acid, malic acid, (-)-L-malic acid, malonic acid, (±)-DL-mandelic acid, methanesulfonic acid, naphthalene-2-sulfonic acid, naphthalene-1,5-disulfonic acid, 1-hydroxyl-2-naphthoic acid, nicotinic acid, nitric acid, oleic acid, orotic acid, oxalic acid, palmitic acid, pamoic acid, phosphoric acid, propionic acid, L-pyroglutamic acid, salicylic acid, 4-aminosalicylic acid, sebacic acid, stearic acid, succinic acid, sulfuric acid, tannic acid, (+)-L-tartaric acid, sulfocyanic acid, p-toluenesulfonic acid, undecylenic acid, pentanoic acid, and acylated amino acid.

In the study of PQQ, the inventors found PQQ is effective in preventing, alleviating or treating patient with primary dysmenorrhea, secondary dysmenorrhea (not according to the claimed invention) and menstrual abnormalities accidentally.

In some embodiments, after being treated with PQQ, at least one of the following effects is achieved for patients: 1. the dysmenorrhea alleviation rate is 100%; 2. the average duration of a menstrual period is reduced from about 2 weeks to about 1 week, and is returned to the normal average duration of a menstrual period; 3. the amount of menstrual blood in a menstrual period is reduced by more than 30% on average, and is substantially returned to the normal average level; and 4. two patients suffering from years of infertility caused by secondary dysmenorrhea are pregnant within a treatment period of 3 months. Details are as following:

### Treatment of patients with primary dysmenorrhea:

Experiment number: 100 people have been diagnosed with primary dysmenorrhea, 50 people in the experimental group and the control group.

Experimental method: administering PQQ orally to the patients of experimental group, administering placebo orally to the patients of control group. The duration of the experiment was 3 months, and menstrual pain was asked and recorded.

### The results of the experiment:

Experimental group: the dysmenorrhea index alleviated by 100% compared to beginning of the experiment.

Control group: the dysmenorrhea index alleviated by less than 20%, compared to beginning of the experiment, and the average degree of alleviation was lower than the experimental group.

| **Proportion of people with alleviation in primary dysmenorrhea (self-assessment)** | | |
|---|---|---|
| Evaluation time | PQQ group (50 people) | Control group (50 people) |
| 1 month | 98% | 12% |
| 3 months | 100% | 16% |

Compared with the control group, the data of experimental group proved that the drug is effective with significant statistical significance for treating primary dysmenorrhea.

### Treatment of patients with secondary dysmenorrhea (for illustrative purposes only):

Experiment number: 100 peoples have been diagnosed with secondary dysmenorrhea, with 50 peoples in the experimental group and the control group each.

Experimental method: administering PQQ orally to the patients of experimental group, administering placebo orally to the patients of control group. The duration of the experiment was 3 months, and menstrual pain and pregnancy were asked and recorded.

### The results of the experiment:

Experimental group: the dysmenorrhea index alleviated by 100% compared to the beginning of the experiment; 2 cases became pregnant.

Control group: the dysmenorrhea index alleviated by less than 5% compared to the beginning of the experiment, and the average degree of alleviation was far lower than the experimental group; no case became pregnant.

| **Proportion of people with alleviation in secondary dysmenorrhea** | | |
|---|---|---|
| Evaluation time | PQQ group (50 people) | Control group (50 people) |
| 1 month | 82% | 8% |
| 3 months | 100% | 4% |

Compared with the control group, the data of experimental group proved that the drug is effective with significant statistical significance for treating secondary dysmenorrhea.

The dysmenorrhea index is determined based on the VAS scoring standard, which means patients self-evaluate the intensity of pain and the degree of psychologically unpleasant experience according to the degree of pain they feel, including:
0 points: no pain;
1-3 points: mild pain;
4-6 points: moderate pain;
7-9 points: severe pain;
10 points: unbearable pain.

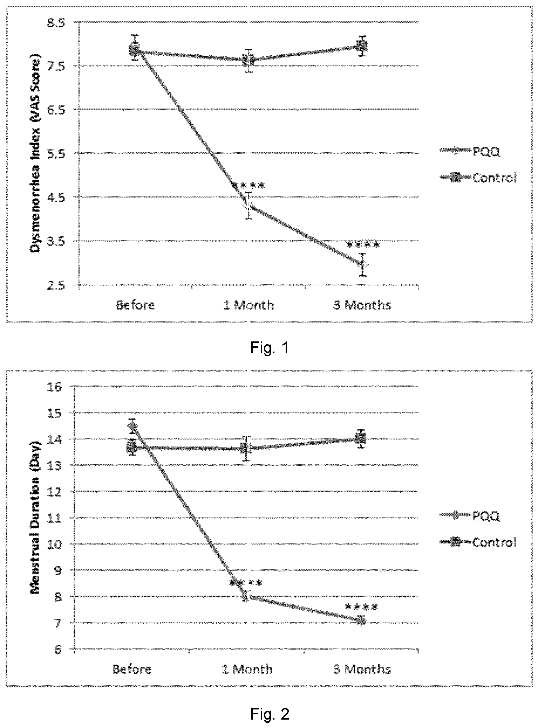

The average dysmenorrhea index changes is shown in Figure 1. According to the figure, the dysmenorrhea index of the PQQ group decreased significantly, while the dysmenorrhea index of the control group did not change significantly. It was proved that secondary dysmenorrhea is significantly alleviating by administering PQQ preparation orally.

### Treatment of patients with abnormal menstruation:

Experiment number: 100 peoples diagnosed with menstrual abnormalities, 50 people in the experimental group and the control group each.

Experimental method: administering PQQ orally to the patients of experimental group, administering placebo orally to the patients of control group. The duration of the experiment was 3 months, and menstrual conditions (duration of menstrual, consumption and weights of sanitary napkin) were measured and recorded.

### The results of the experiment:

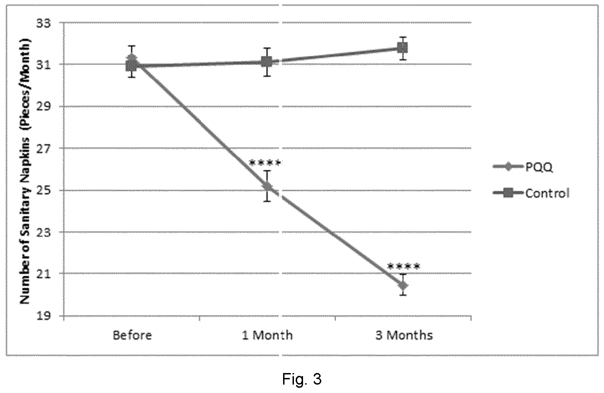

The experimental results are shown in Figure 2 and Figure 3. As shown in Figure 2 and Figure 3, in the experimental group: the menstrual duration: the average monthly menstrual bleeding duration was reduced from about 2 weeks to about 1 week compared to beginning of the experiment; the amount of menstrual bleeding: the average amount of menstrual bleeding decreased by more than 30%; in the control group: compared with the beginning of the experiment, menstrual duration: no significant alleviation; the amount of menstrual bleeding : no significant alleviation.

Compared with the control group, the data of experimental group proved that the drug is effective with significant statistical significance for treating menstrual abnormalities.

## Claims

1. Pyrroloquinoline quinone or derivatives thereof for use in a method of preventing, alleviating or treating a disease selected from:
- primary dysmenorrhea; and
- menstrual abnormalities selected from an abnormal increase in the amount of menstrual bleeding, and/or an abnormal increase in the duration of menstrual bleeding,
wherein said derivative of pyrroloquinoline quinone is a pharmaceutically or food acceptable salt, C2-C6 ester, amide, hydrate, crystal, co-crystal or solvate.

2. Pyrroloquinoline quinone or derivatives thereof for use according to claim 1, wherein:
said abnormal increase in the amount of menstrual bleeding refers to that the amount of menstrual bleeding is higher than 50% of the normal amount of menstrual bleeding in women; and
said abnormal increase in the duration of menstrual bleeding refers to the duration of menstrual bleeding is no less than 8 days.

3. Pyrroloquinoline quinone or derivatives thereof for use according claim 1, wherein the oral dosage based on pyrroloquinoline quinone is:
about 5 mg/day to about 10 mg/day for preventing of primary dysmenorrhea;
about 15 mg/day to about 20 mg/day for alleviating or treating mild dysmenorrhea;
about 20 mg/day to about 30 mg/day for alleviating or treating moderate dysmenorrhea;
about 25 mg/day to about 100 mg/day for alleviating or treating severe dysmenorrhea;
about 20 mg/day to about 50 mg/day for alleviating or treating menstrual abnormalities.

4. Pyrroloquinoline quinone or derivatives thereof for use according claim 1, wherein said composition is a food, a nutraceutical or a medicine.

5. Pyrroloquinoline quinone or derivatives thereof for use according claim 1, wherein said composition further comprising pharmaceutical or food acceptable excipients.

6. Pyrroloquinoline quinone or derivatives thereof for use according to claim 5, wherein said excipient is selected from at least one of carriers, solvents, antioxidants, and preservatives.

## Patentansprüche

1. Pyrrolochinolinchinon oder Derivate davon zur Verwendung bei einer Methode zur Vorbeugung, Linderung oder Behandlung einer Krankheit, ausgewählt aus:
- primäre Dysmenorrhö; und
- Menstruationsstörungen, ausgewählt aus einem abnormalen Anstieg der Menge der Menstruationsblutung und/oder einem abnormalen Anstieg der Dauer der Menstruationsblutung, wobei das Derivat von Pyrrolochinolinchinon ein pharmazeutisches oder für Lebensmittel zulässiges Salz, ein C2-C6-Ester, ein Amid, ein Hydrat, ein Kristall, ein Co-Kristall oder ein Solvat ist.

2. Pyrrolochinolinchinon oder Derivate davon zur Verwendung nach Anspruch 1, wobei:
der abnormale Anstieg der Menstruationsblutung bedeutet, dass die Menge der Menstruationsblutung mehr als 50 % der normalen Menge der Menstruationsblutung bei Frauen beträgt; und
der abnormale Anstieg der Dauer der Menstruationsblutung sich auf die Dauer der Menstruationsblutung von nicht weniger als 8 Tagen bezieht.

3. Pyrrolochinolinchinon oder Derivate davon zur Verwendung nach Anspruch 1, wobei die orale Dosierung auf Basis von Pyrrolochinolinchinon Folgendes beträgt:
etwa 5 mg/Tag bis etwa 10 mg/Tag zur Vorbeugung einer primären Dysmenorrhö;
etwa 15 mg/Tag bis etwa 20 mg/Tag zur Linderung oder Behandlung leichter Dysmenorrhö;
etwa 20 mg/Tag bis etwa 30 mg/Tag zur Linderung oder Behandlung mittelschwerer Dysmenorrhö;
etwa 25 mg/Tag bis etwa 100 mg/Tag zur Linderung oder Behandlung schwerer Dysmenorrhö;
etwa 20 mg/Tag bis etwa 50 mg/Tag zur Linderung oder Behandlung von Menstruationsstörungen.

4. Pyrrolochinolinchinon oder Derivate davon zur Verwendung nach Anspruch 1, wobei die Zusammensetzung ein Lebensmittel, ein Nahrungsergänzungsmittel oder ein Arzneimittel ist.

5. Pyrrolochinolinchinon oder Derivate davon zur Verwendung nach Anspruch 1, wobei die Zusammensetzung ferner pharmazeutische oder für Lebensmittel zulässige Hilfsstoffe umfasst.

6. Pyrrolochinolinchinon oder Derivate davon zur Verwendung nach Anspruch 5, wobei der Hilfsstoff aus mindestens einem der Träger, Lösungsmittel, Antioxidantien und Konservierungsmittel ausgewählt ist.

## Revendications

1. Pyrroloquinoline quinone ou ses dérivés pour une utilisation dans un procédé de prévention, de soulagement ou de traitement d'une maladie choisie parmi :
- la dysménorrhée primaire ; et
- des anomalies menstruelles choisies parmi une augmentation anormale de la quantité de saignements menstruels, et/ou une augmentation anormale de la durée de saignements menstruels, dans laquelle ou lesquels ledit dérivé de pyrroloquinoline quinone est un sel pharmaceutiquement ou alimentairement acceptable, ester en C2-C6, amide, hydrate, cristal, co-cristal ou solvate.

2. Pyrroloquinoline quinone ou ses dérivés pour une utilisation selon la revendication 1, dans laquelle ou lesquels :
ladite augmentation anormale de la quantité de saignements menstruels fait référence au fait que la quantité de saignements menstruels est supérieure à 50 % de la quantité normale de saignements menstruels chez les femmes ; et
ladite augmentation anormale de la durée des saignements menstruels fait référence au fait que la durée des saignements menstruels n'est pas inférieure à 8 jours.

3. Pyrroloquinoline quinone ou ses dérivés pour une utilisation selon la revendication 1, dans laquelle ou lesquels la posologie orale à base de pyrroloquinoline quinone est :
environ 5 mg/jour à environ 10 mg/jour pour prévenir la dysménorrhée primaire ;
environ 15 mg/jour à environ 20 mg/jour pour soulager ou traiter une dysménorrhée légère ;
environ 20 mg/jour à environ 30 mg/jour pour soulager ou traiter une dysménorrhée modérée ;
environ 25 mg/jour à environ 100 mg/jour pour soulager ou traiter une dysménorrhée sévère ;
environ 20 mg/jour à environ 50 mg/jour pour soulager ou traiter les anomalies menstruelles.

4. Pyrroloquinoline quinone ou ses dérivés pour une utilisation selon la revendication 1, dans laquelle ou lesquels ladite composition est un aliment, un nutraceutique ou un médicament.

5. Pyrroloquinoline quinone ou ses dérivés pour une utilisation selon la revendication 1, dans laquelle ou lesquels ladite composition comporte en outre des excipients pharmaceutiquement ou alimentairement acceptables.

6. Pyrroloquinoline quinone ou ses dérivés pour une utilisation selon la revendication 5, dans laquelle ou lesquels ledit excipient est choisi parmi au moins l'un parmi des supports, des solvants, des antioxydants et des conservateurs.
